# EUROPEAN PATENT APPLICATION

(11) **EP 3 798 300 A1**
(43) Date of publication of application: **31.03.2021**
(21) Application number: 19199362.5
(22) Date of filing: 24.09.2019
(51) Int. Cl.: C12N 5/00, C07K 14/47, C08C 19/00

(54) **CELL CULTURING MATERIALS**

(71) Applicant: Koninklijke Philips N.V., 5656 AG Eindhoven (NL)
(72) Inventor: BECKERS, Lucas Johannes Anna Maria, 5656 AE Eindhoven (NL); VAN GIJSEL, Thomas Johannes, 5656 AE Eindhoven (NL)
(74) Representative: de Haan, Poul Erik

(57) **Abstract**

A material for culturing cells is disclosed. The material contains a bulk-modified elastomer having a Shore hardness (DIN EN ISO 868) in a range of Shore00 20 to Shore A 80 and comprising a plurality of fatty acid moieties covalently bound to the elastomer bulk, wherein the carboxylic acid groups of said moieties are available on an external surface of said material to provide said binding, and wherein the bulk-modified elastomer is obtained by forming a composition comprising a vinyl-functionalized or a hydride-functionalized elastomer or at least one precursor thereof, a free of saponified unsaturated fatty acid in a range of 0.5 - 5 % by weight of the total weight of the a vinyl-functionalized or a hydride-functionalized elastomer or at least one precursor thereof and a cross-linking catalyst in a mold having a polar inner surface; and bulk-modifying the vinyl-functionalized or the hydride-functionalized elastomer by covalently binding the free or saponified unsaturated fatty acid to the elastomer bulk in said mold by a cross-linking reaction between a vinyl group or a hydride group of the elastomer and an unsaturated carbon-carbon bond of the unsaturated fatty acid to obtain the material. Also disclosed are a fluidic device module and fluidic device, a cell culturing method and a drug testing method.

## Description

### FIELD OF THE INVENTION

The present invention relates to an elastomer material for culturing harvested cells.

The present invention further relates to a fluidic device module and fluidic device comprising such an elastomer material.

The present invention further relates to a method of culturing cells using such an elastomer material.

The present invention further relates to a drug testing method with a fluidic device module comprising such an elastomeric material.

### BACKGROUND OF THE INVENTION

In-vitro testing of mammalian cells or tissue is an important technique to obtain clinically important information of the mammalian material under investigation. For example, biopsied mammalian cell or tissue material may be subjected to such testing in order to determine anomalies or disease in such mammalian material or to expose diseased mammalian material to drugs, e.g. experimental drugs, to monitor the response of the diseased mammalian material to such exposure. This approach for example is frequently used in oncological procedures. This can provide important insights in how a disease in an individual can be effectively treated without having to expose the individual to a range of potentially effective drugs, which can be undesirable for a number of reasons, such as drug toxicity. In addition, the efficacy of experimental drugs for existing diseases for which no established satisfactory drug treatment is yet available may be tested in this manner. There are many other well-known reasons for deploying such in-vitro tests.

A common approach to such in-vitro testing is to immobilize the mammalian material in a fluidic device, which is sometimes referred to as an organ-on-chip. In such an approach, the mammalian material is typically immobilized on a membrane separating two fluidic channels of the fluidic device, with a first channel being used to feed the mammalian material and the second channel being used to expose the mammalian material to a chemical compound or composition of interest such as a drug treatment, e.g. to test the efficacy and/or toxicity of the drug as previously explained. The fluidic device, or at least its membrane, may be made of an elastomer such that the fluidic device may be diced or sliced in order to obtain a slice of the membrane including the mammalian material for evaluation purposes, e.g. to evaluate the reaction of the mammalian material to the exposure to the chemical compound or composition of interest.

A challenge in such in-vitro testing is to ensure that the mammalian material, e.g. cells or tissue, are stabilized and develop normally on the membrane of the fluidic device such that they live for the duration of the test procedure. This is particularly challenging in oncology procedures where experiments can be rather lengthy. To this end, the mammalian material may be stabilized using a biocompatible material such as fibronectin, which ensures that the mammalian cells will keep multiplying by providing the cells with a chemical environment mimicking that of natural tissue.

A common approach is to coat a well cell plate with a polymer having carboxylic acid groups, as such groups can (covalently) bond to the fibronectin, thereby facilitating the stabilization of the mammalian cells on the membrane within the fluidic device as the fibronectin makes the polymer bioconnective. However, this approach is not without its disadvantages. Firstly, the fluidic streams through the fluidic device may partially remove the aforementioned polymer, which compromises the ability to evaluate the mammalian material at a desired point in time due to the fact that at least some of this material may have been lost. In another approach, the membrane is treated with UV or plasma to generate binding sites for the fibronectin on the membrane. However, this approach has the drawback that it is rather difficult to avoid an inhomogeneous distribution of such binding sites, which again hampers evaluation of testing results.

Secondly, membranes comprising such a biocompatible coating are notoriously difficult to manufacture, which makes the manufacture of such fluidic devices rather costly and cumbersome. For example, such membranes typically need to be manufactured using thin-film technology, which is expensive, and need to be integrated within the fluidic device without leakage, which is far from straightforward. In addition, where the membrane itself is not made of a biocompatible material the membrane typically needs to be provided with a plurality of cell-sized holes with a small pitch to facilitate cell growth, which is also difficult to achieve.

An example of the microfabrication of such a human organ-on-chip based on thin film techniques is disclosed by Dongeun Huh et al. in Nature Protocols, Vol. 8, No. 11, 2013, pages 2135-2157. In this protocol, micro-engineering is used to fabricate a multilayered microfluidic device containing two parallel elastomeric micro-channels separated by a thin porous flexible membrane along with two full-height, although vacuum chambers on either side, which device takes approximately 3 ½ days to produce. The total microfabrication procedure includes over 100 steps, with a significant number of them being critical steps. This clearly demonstrates the complexity of such manufacturing procedures.

An improvement over such laborious approaches is disclosed in WO 2018/021906 A1, which discloses a silicon-based (PDMS) fluidic device that can be made biocompatible using a coating of e.g. collagen. Such a device may be manufactured in a few manufacturing steps only, but still suffers from the drawback that it needs coating with a biocompatible material, which as explained above can erode from the polymer surface when exposed to fluid streams, thus leading to the unwanted loss of cell material from the fluidic device surface. This is addressed in WO 2018/021906 A1 by plasma-treating the surface of the device and subsequently functionalizing the surface with 3-aminopropyltriethoxysilane and glutaraldehyde prior to coating the functionalized surface with collagen and removing unbound collagen by flushing the microchannels of the fluidic device with EGM-2 (Endothelial Cell Growth Medium-2 BulletKit as marketed by the Lonza corporation). Hence, a substantial number of processing steps are still required to provide a biocompatible fluidic device.

A solution to this problem is disclosed by the applicant in WO 2019/015988 A1. In this application, a material for binding to a cell culturing protein is disclosed. The material contains a bulk-modified elastomer comprising a plurality of fatty acid moieties covalently bound to the elastomer bulk, wherein the carboxylic acid groups of said moieties are available to provide said binding on the surface of an object formed from the bulk-modified elastomer by bulk-modifying the elastomer with the fatty acid moieties in a mold in the shape of the object and having a polar inner surface such that the hydrophilic carboxylic acid groups of the fatty acid moieties are attracted to this polar inner surface. It has been demonstrated in this prior application that a biocompatible material such as fibronectin may be covalently bound to the surface carboxylic acid groups of the bulk-modified elastomer whilst retaining its cell culturing properties.

Nevertheless, it is desirable to further simplify the culturing of cells on such a bulk-modified elastomer.

### SUMMARY OF THE INVENTION

The present invention seeks to provide an elastomeric material for culturing harvested cells directly onto the elastomeric material.

The present invention further seeks to provide a fluidic device module and fluidic device comprising such an elastomeric material.

The present invention further seeks to provide a method of culturing cells such an elastomeric material.

The present invention further seeks to provide a drug testing method using such a fluidic device.

According to an aspect, there is provided a material for culturing cells, the material containing a bulk-modified elastomer having a Shore hardness (DIN EN ISO 868) in a range of Shore00 20 to Shore A 80 and comprising a plurality of fatty acid moieties covalently bound to the elastomer bulk, wherein the carboxylic acid groups of said moieties are available on an external surface of said material to provide said binding, and wherein the bulk-modified elastomer is obtained by forming a composition comprising a vinyl-functionalized or a hydride-functionalized elastomer or at least one precursor thereof, a free of saponified unsaturated fatty acid in a range of 0.5 - 5 % by weight of the total weight of the a vinyl-functionalized or a hydride-functionalized elastomer or at least one precursor thereof and a cross-linking catalyst in a mold having a polar inner surface; and bulk-modifying the vinyl-functionalized or the hydride-functionalized elastomer by covalently binding the free or saponified unsaturated fatty acid to the elastomer bulk in said mold by a cross-linking reaction between a vinyl group or a hydride group of the elastomer and an unsaturated carbon-carbon bond of the unsaturated fatty acid to obtain the material.

It has surprisingly been found that a relatively soft elastomer, i.e. having a Shore hardness in a range of a range of Shore00 20 to Shore A 80 (or Shore A 2-80) as determined using the measurement method specified in the DIN EN ISO 868 standard, when bulk modified with an unsaturated fatty acid as previously disclosed in WO 2019/015988 A1 can act as a biocompatible material onto which cellular material can be directly cultured without the need for an extracellular matrix such as fibronectin when the amount of unsaturated fatty acid is in a range of 0.5 - 5 % by weight (wt%) based on the total weight of the elastomer or its at least one precursor. Without wishing to be bound by theory, it is believed that where the concentration of unsaturated fatty acid is below 0.5 wt%, the density of carboxylic acid groups or carboxylate groups in case of a saponified unsaturated fatty acid is too low to make the external surface of the elastomer onto which the cells are to proliferate sufficiently hydrophilic to achieve suitable proliferation conditions, whereas when this concentration is above 5wt%, some unreacted unsaturated fatty acid may leach from the bulk-modified elastomer and poison the cells adhered to its external surface. Consequently, it is expected that the bulk-modified elastomer has a covalently bound fraction of free or saponified fatty acid groups in a range of 0.5 - 5 wt% based on the total weight of the elastomer given that in this range a full conversion of the cross-linking reaction between the elastomer (or its one or more precursors) and the unsaturated fatty acid in free or saponified form is expected.

The bulk-modified elastomer is chosen to have a hardness in a range of Shore 00 20 to Shore A 80 to ensure that the elastomer is sufficiently flexible to promote cell proliferation, as it for instance is well-known per se that softness of a cell carrier material is essential for growth (and differentiation) of stem cells due to the fact that tissues grown from stem cells want to move, e.g. (heart) muscle, lung, gut and blood vessel tissues. Some organs need to grow in a movable carrier like heart muscles. Hence, where the bulk-modified elastomer material has a Shore A hardness in excess of 80, the material becomes insufficiently stretchable to facilitate the desired cell proliferation directly onto its functionalized surface, in which case the use of a scaffold or extracellular matrix material would be required to provide the developing cell culture with its desired flexibility.

Furthermore, the unsaturated fatty acid used in the bulk modification of the elastomer may be used in free or saponified form, such as for example as a sodium salt. The use of the unsaturated fatty acid in saponified form has the advantage that the risk of catalyst poisoning by the unsaturated fatty acid protons is avoided, whilst it has surprisingly been found that cell culture will also proliferate onto the external surface of a bulk-modified elastomer carrying carboxylate groups with a suitable counter ion such as Na⁺ or K⁺. Hence, this has the further advantage that the saponification does not have to be reversed prior to using the bulk-modified elastomer for cell culturing purposes.

Hence, in this manner a material for cell culturing can be manufactured in a small number of steps, e.g. a single step in some embodiments, by combining the elastomer and the fatty acid in a coating (spin coating, dip coating, spray coating, dispensing) or an injection molding process in which the cross-linking of the elastomer with the fatty acid carbon-carbon double bond can be achieved without significant epoxidation of the fatty acid carbon-carbon double bond due to the limited exposure to ambient oxygen in the spin coating or injection molding process. Consequently, a material is provided in which the elastomer is bulk modified with fatty acid moieties in which the carboxylic acid or carboxylate groups of the (saponified) fatty acid are available to directly bind to harvested cells. Moreover, by controlling the curing process and/or the properties of the mold in which the material is formed, such carboxylic acid or carboxylate groups are also present on an outer surface of the material, thereby providing a substantially homogeneous distribution of carboxylic acid or carboxylate groups on such outer surface, which makes the material particularly suitable for use as a membrane material for a fluidic device, as each cross-section of the material will exhibit the same surface properties, in contrast to membrane materials onto which an anchor for a biocompatible material needs to be grafted or otherwise formed as previously explained. In addition, because typically only a fraction of the elastomer carbon-carbon double bonds are consumed in such a cross-linking reaction, the inventive material retains the elastomeric properties of the elastomer, which adds to the suitability of the inventive material for use in fluidic devices and facilitates the slicing or otherwise cutting of the inventive material for investigative purposes.

Preferably, each of the free or saponified fatty acid moieties is covalently bound to the elastomer bulk through a cross-linking reaction between a vinyl functional group or a hydride functional group of the elastomer and an unsaturated carbon-carbon bond of an unsaturated fatty acid to ensure that the number of carboxylic acid or carboxylate groups available for binding to the biocompatible material can be optimized.

The unsaturated fatty acid may be any suitable unsaturated fatty acid. In an example embodiment, the unsaturated fatty acid is selected from myristoleic acid, palmitoleic acid, sapienic acid, oleic acid, elaidic acid, vaccenic acid, linoleic acid, linoeladic acid, α-linolenic acid, arachidonic acid, eicospaentaenoic acid, erucic acid, undodecanoic acid and docosahexaenoic acid. Linoleic acid is specifically mentioned.

Similarly, the elastomer may be any suitable elastomer. In example embodiments, the elastomer comprises a polybutadiene backbone or a silicone backbone. Where the elastomer comprises a silicone backbone, at least a fraction of the carboxylic acid groups within the silicone backbone may be saponified as saponification of the carboxylic acid groups prior to the cross-linking between the unsaturated fatty acid molecules and the elastomer molecules may be required to prevent the cross-linking catalyst from being deactivated by the protons of the carboxylic acid groups of the unsaturated fatty acid molecules. Equally, the polybutadiene may be cross-linked with a saponified unsaturated fatty acid as the cell culturing properties of the bulk-modified material are not significantly affected by saponification of the unsaturated fatty acid as previously explained.

In a set of embodiments, the composition comprises the free or saponified unsaturated fatty acid in a range of 0.5 - 2 % by weight of the total weight of the a vinyl-functionalized or a hydride-functionalized elastomer or at least one precursor thereof, as it has been found that particularly good cell proliferation is achieved when the amount of free or saponified unsaturated fatty acid to be incorporated into the bulk of the elastomer is within this range. For example, excellent cell proliferation is achieved directly onto the external surface of the bulk-modified elastomer when the composition comprises the free or saponified unsaturated fatty acid in an amount of about 1 % by weight of the total weight of the a vinyl-functionalized or a hydride-functionalized elastomer or at least one precursor thereof.

According to yet a further aspect, there is provided a fluidic device module including a flow channel extending over a membrane, the membrane comprising the inventive material according to any of the herein described embodiments. Such a fluidic device module may comprise a first major surface comprising a first recessed structure defining a first flow channel; a second major surface opposing the first major surface and comprising a second recessed structure defining a second flow channel; with the membrane separating the first flow channel from the second flow channel. In a preferred embodiment, the fluidic device module is a monolithic fluidic device module, which has the advantage that the device module can be manufactured in a small number of processing steps, e.g. a single processing step, by injection molding.

The membrane may comprise a plurality of holes or grooves extending through the membrane. Such holes or grooves preferably have cell-size dimensions to prevent cells from passing through the membrane.

In an embodiment, the first flow channel and the second flow channel are accessible through respective septums, such that the separate flow channels may be individually accessed without cross-contamination risk.

According to yet a further aspect, there is provided a fluidic device comprising the fluidic device module of any of the herein described embodiments and a pair of cover plates to fluidly seal the fluidic device module. The cover plates may be arranged such that one of said cover plates covers the first major surface, thereby sealing the first fluidic channel and the other of said cover plates covers the second major surface, thereby sealing the second fluidic channel. Such a fluidic device can be manufactured in a straightforward manner, as the fluidic device module may be formed in a small number of processing steps as previously explained, and is robust against leakage due to the flexible nature of the fluidic device module.

According to yet a further aspect, there is provided a method of culturing cells, comprising providing a molded article formed from the bulk-modified elastomer material of any of the herein described embodiments, said article comprising the external surface of the material as one of its exposed surfaces; and culturing said cells directly on said exposed surface. In accordance with this method, cells can proliferate directly onto the exposed surface of such an article, e.g. a fluid device module as previously described, without the need for attaching an extracellular matrix such as fibronectin or the like to the exposed surface of the article, thereby significantly simplifying the cell culturing process.

According to still a further aspect, there is provided a drug testing method comprising providing a fluidic device module according to an embodiment; applying harvested cells to a surface of the membrane of the fluidic device module; culturing the harvested cells on said surface; forming a fluidic device with the prepared fluidic device module; feeding the cultured cells through one of the pair of flow channels of the fluidic device; exposing the cultured cells to a drug to be tested through the other of the pair of flow channels of the fluidic device; and monitoring the response of the cultured cells to the drug to be tested. Such a drug testing method can be deployed in a simple and straightforward manner, in particular in terms of preparation of the fluidic device to be used in such a drug testing method, thereby providing a significant simplification of existing drug testing methods where such fluidic devices are typically very cumbersome to prepare.

The monitoring of the response of the cultured cells to the drug to be tested may comprise immobilizing and fixating the cultured cells within the fluid device and slicing the fluid device module to obtain a slice for microscopic evaluation, said slice comprising at least a portion of the immobilized and fixated cultured cells. In accordance with the teachings of the present invention, the generation of such slices for microscopic evaluation no longer is critical or subject to variable results due to the fact that the cultured cells are homogeneously distributed across the membrane surface of the fluidic device module owing to the bulk-modification of the elastomer with a free or saponified unsaturated fatty acid as previously explained.

Alternatively, in a preferred embodiment, the monitoring of the response of the cultured cells may be performed within the assembled fluid device using confocal microscopy. This facilitates the real-time monitoring of such responses.

### BRIEF DESCRIPTION OF THE DRAWINGS

Embodiments of the invention are described in more detail and by way of non-limiting examples with reference to the accompanying drawings, wherein:
FIG. 1 schematically depicts the bulk modification of an elastomer with an unsaturated fatty acid according to an embodiment;
FIG. 2 schematically depicts a perspective view of a fluidic device module according to an embodiment;
FIG. 3 schematically depicts another perspective view of a fluidic device module according to an embodiment;
FIG. 4 schematically depicts a perspective view of a fluidic device module according to another embodiment;
FIG. 5 schematically depicts an exploded perspective view of a fluidic device according to an embodiment;
FIG. 6 schematically depicts an exploded perspective view of a fluidic device according to another embodiment;
FIG. 7 schematically depicts an exploded perspective view of a fluidic device according to yet another embodiment;
FIG. 8 schematically depicts an exploded perspective view of a fluidic device according to yet another embodiment;
FIG. 9 is a microscopic image of a cell culture developed on a bulk-modified elastomer according to an embodiment;
FIG. 10; is a microscopic image of a cell culture developed on an elastomer without such bulk modification;
FIG. 11 is a microscopic image of a cell culture on a bulk-modified elastomer according to another embodiment after developing for 1 day;
FIG. 12 is a microscopic image of a cell culture on a bulk-modified elastomer according to another embodiment after developing for 2 days; and;
FIG. 13 is a microscopic image of a cell culture on a bulk-modified elastomer according to another embodiment after developing for 7 days.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

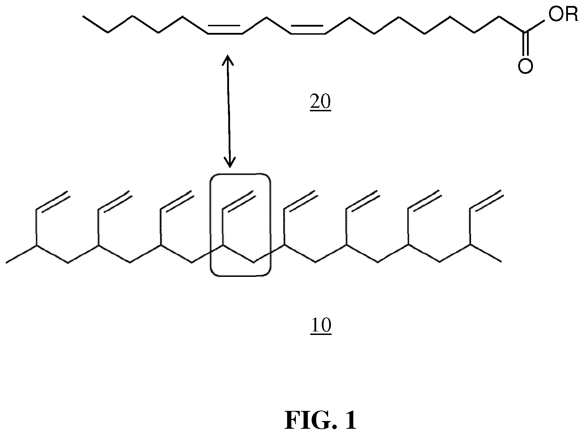

FIG. 1 schematically depicts the cross-linking reaction for producing the bulk-modified elastomer material according to embodiments of the present invention. In this cross-linking reaction, a vinyl-functionalized elastomer 10 (from here on simply referred to as elastomer) is cross-linked to an unsaturated fatty acid 20 by a cross-linking reaction between one of the carbon-carbon double bonds of the elastomer 10 and one of the carbon-carbon double bonds of the unsaturated fatty acid 20, which cross-linking reaction may be catalyzed using an appropriate cross-linking catalyst. It has surprisingly been found by the present inventors that such a cross-linking reaction can provide a bulk-modified elastomer in which the carboxylic acid groups of at least a fraction of the unsaturated fatty acid molecules 20 having engaged in the cross-linking reaction with the elastomer 10 are presented on an external surface of the bulk-modified elastomer, such that these carboxylic acid groups are available for stabilizing cell cultures directly on the external surface of the bulk-modified elastomer 10. To this end, the elastomer 10 should be sufficiently soft or flexible to facilitate cell-induced movement. For this reason, the elastomer 10 typically has a Shore hardness as determined in accordance with the DIN EN ISO 868 standard ranging from Shore 00 20 (i.e. Shore A 2) to Shore A 80, i.e. a Shore A hardness of 2-80. Suitable elastomers include polyenes such as polybutadiene and silicones.

The unsaturated fatty acid 20 may be a free unsaturated fatty acid, in which case R = H or may be a saponified fatty acid, in which case R = Na⁺ or K⁺ (and the COO group is a COO⁻ or carboxylate anionic group) for example. Such saponification may be achieved in any suitable manner, e.g. using a concentrated NaOH or KOH solution. As such saponification reactions are well-known per se, this will not be explained in further detail for the sake of brevity only. Importantly, the saponification of the unsaturated fatty acids does not need to be reversed as it has been surprisingly found that cell proliferation on the external surface of the bulk-modified elastomer 10 also takes place when this surface is functionalized with saponified carboxylic acid groups, i.e. carboxylate groups having a counter ion such as a Na⁺ or K⁺ counter ion, as will be explained in further detail below.

The presence of the carboxylic acid or carboxylate groups on the external surface of the bulk-modified elastomer 10 may be considered surprising given that the elastomer 10 can be non-polar in character, in particular when the elastomer 10 is dissolved in a non-polar solvent. In such a non-polar environment, the unsaturated fatty acid molecules 20 tend to form micelles in which the carboxylic acid moieties of these molecules are turned inwardly within such micelles, which typically leads to such carboxylic acid moieties ending up within the bulk of the modified elastomer. In addition, it is well-known per se that the carbon-carbon double bonds of such unsaturated fatty acids 20 are prone to rapid epoxidation in the presence of oxygen, which reduces the ability of such compounds to engage in cross-linking reactions with the elastomer 10.

Embodiments of the present invention are based on the insight that if a reaction mixture including the elastomer 10 and the unsaturated fatty acid 20 is brought into contact with a polar surface, this promotes orientation of the carboxylic acid groups of the fatty acid molecules along such a polar surface such that an external surface of the bulk-modified elastomer material exhibits a high density and homogeneous distribution of these carboxylic acid groups. Moreover, it has been found that when bulk modifying the elastomer 10 in this manner in an injection molding process at elevated temperatures, e.g. temperatures in a range of 120-240°C, epoxidation of the carbon-carbon double bonds of the unsaturated fatty acid molecules 20 does not significantly interfere with the bulk modification of the elastomer 10, i.e. does not significantly inhibit the cross-linking reaction between the unsaturated fatty acid molecules 20 and the elastomer 10. Without wishing to be bound by theory, it is believed that in such injection molding processes, molecular oxygen is largely absent from the reaction mixture within the injection molding apparatus, thereby suppressing the unwanted epoxidation of these carbon-carbon double bonds. The cross-linking reaction between the elastomer 10 and the unsaturated fatty acid 20 may involve the formation of a covalent bond by a reaction between a hydride functional group of the elastomer 10 and the carbon-carbon double bond of the unsaturated fatty acid 20, such as for example in the case of hydride-functionalized silicones, as will be explained in further detail below.

In order to obtain an elastomer 10 onto which cells can be directly cultured, the elastomer 10 is bulk-modified with an amount of the unsaturated fatty acid 20 in free or saponified form in a range of 0.5 - 5 % by weight of the total weight of the elastomer 10 or the total weight of the one or more precursors of the elastomer 10. If a smaller amount of the unsaturated fatty acid 20 in free or saponified form is used, it has been found that cells will not proliferate onto the bulk-modified elastomer 10, which may be due to the surface of the bulk-modified elastomer 10 onto which the cells are seeded being too hydrophobic in nature. On the other hand, if a larger amount of the unsaturated fatty acid 20 in free or saponified form is used, the cells also fail to proliferate on the surface of the bulk-modified elastomer 10, which may be caused by unreacted unsaturated fatty acid 20 in free or saponified form poisoning the cell culture. Moreover, it has been found that when the amount of unsaturated fatty acid 20 in free or saponified form is in this range, the flexibility or hardness of the elastomer 10 is not significantly altered by the bulk modification with the unsaturated fatty acid 20 in free or saponified form.

Any suitable elastomer having the desired Shore hardness may be used for this purpose. For example, the elastomer may be a homopolymer, a copolymer, a block copolymer, a terpolymer, a block terpolymer and so on. A particularly suitable elastomer may be selected from polyenes such as polybutadiene. Where polybutadiene is used, the polybutadiene may be made using any suitable polymerization process. Particularly preferred is a polybutadiene formed in a Nd or Li-catalyzed polymerization reaction, as it is well-known per se that such polybutadiene has a low degree of branching (typically below 5%) and a low degree of polydispersity (Mw/Mn) of around 2. Li-catalyzed polybutadiene is particularly preferred due to its high degree of 1,2-vinyl content (about 11%). However, other types of polybutadiene, e.g. polybutadiene obtained from a Co, Ni or Ti-catalyzed polymerization reaction may be used instead. Where a polyene such as polybutadiene is used as the elastomer 10, the cross-linking reaction between the elastomer 10 and the unsaturated fatty acid 20 may be catalyzed using a peroxide catalyst. In another advantageous embodiment, a Li-catalyzed polybutadiene is used having a degree of branching in a range of 5-15% as it has been found that when the degree of branching of the polybutadiene is in this range, the reactivity of the polybutadiene with the unsaturated fatty acid 20 is improved. It is noted for the sake of completeness that it is well-known per se how to control the degree of branching when synthesizing polybutadiene such that this will not be further explained for the sake of brevity only.

Another class of elastomer is that are specifically mentioned is silicones (polysiloxanes) for injection molding. Such silicones may comprise a PDMS backbone including vinyl moieties to facilitate cross-linking through Pt-catalyzed addition reactions, e.g. with (poly methyl) hydrogen siloxanes. Instead of or in addition to such cross-linking with (poly methyl) hydrogen siloxanes, the vinyl-functionalized PDMS backbone may be cross-linked with unsaturated fatty acids 20. Such silicones may be formed by a cross-linking reaction between a vinyl-functionalized linear or branched silicone monomer or oligomer, e.g. a T-branched or Q-branched silicone monomer or oligomer and a linear hydride-functionalized silicone monomer or oligomer, between a hydride-functionalized linear or branched silicone monomer or oligomer, e.g. a T-branched or Q-branched silicone monomer or oligomer and a linear vinyl-functionalized silicone monomer or oligomer or mixtures of vinyl-functionalized and hydride-functionalized linear or branched silicone monomers or oligomers, e.g. a T-branched or Q-branched silicone monomer or oligomer and/or a mixture of linear hydride-functionalized and vinyl-functionalized silicone monomer or oligomer.

For example, the two-component silicone may be formed by a cross-linking reaction between a linear component 1 and a linear component 2, which each may correspond to the general formula below:

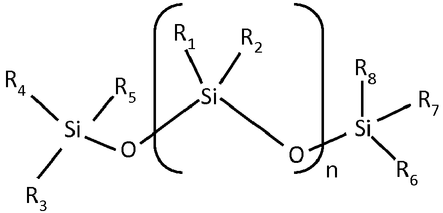

In component 1, R1 and R2 are individually selected from C1-C3 alkyl,R3-R8 are individually selected from C1-C3 alkyl and vinyl, with the proviso that at least one of R3-R5 and at least one of R6-R8 is vinyl. Preferably, at least three of R3-R8 are vinyl. In all embodiments, n may be in the range of 100 - 200,000. In a specific embodiment of component 1, each of the groups R1-R8 that is an alkyl group is a methyl group, i.e. the component 1 is vinyl-functionalized PDMS having terminal vinyl groups.

In component 2, R1 is hydrogen, R2 = C1-C3 alkyl and R2-R8 are individually selected from C1-C3 alkyl or hydrogen, with the proviso that at most one of R3-R5 and at most one of R6-8 is hydrogen, and and n may have any suitable value, such as n = 3-1,000 or more specifically n = 3-10. In a specific embodiment of component 2, none of the groups R3-R8 are hydrogen. In another specific embodiment of component 2, each of R2-R8 are methyl.

Component 2 typically acts as a cross-linking agent for component 1. Such cross-linking reactions, which are typically Pt-catalyzed, are well-known per se, see for example WO 2009/147602 A2 and are therefore not explained in further detail for the sake of brevity. Any suitable silicone elastomer may be used. It should be understood that when cross-linking such silicones in the presence of an unsaturated fatty acid 20, the covalent bond between the silicone and the unsaturated fatty acid 20 alternatively may be formed by a reaction between a hydride functional group of the (cross-linked) silicone and the unsaturated fatty acid 20 as suggested in the below reaction mechanism:

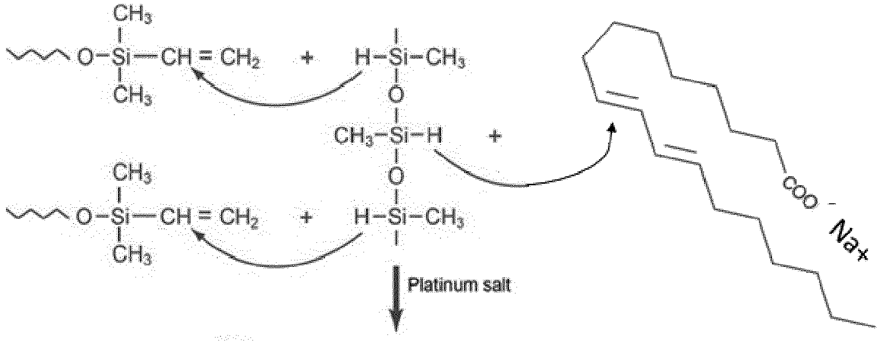

In order to prevent catalyst inhibition by the protons of the carboxylic acid groups of the unsaturated fatty acids 20 during such cross-linking reactions, the unsaturated fatty acid may be used in a saponified form in the cross-linking reaction with a silicone elastomer, e.g. as the sodium salt of such an unsaturated fatty acid. The cross-linking reaction product subsequently may be treated with a strong protic acid, e.g. HCl or the like, in order to reinstate the carboxylic acid groups on the surface of the bulk-modified elastomer, although this is not necessary in case the bulk-modified elastomer is to be used for cell culturing directly on its surface, given that it has been demonstrated that cells will also proliferate when the polar surface groups are carboxylate anions, i.e. saponified carboxylic acid groups. Any suitable type of polysiloxane may be used for this purpose.

Alternatively, a silicone backbone such as a (poly methyl) hydrogen siloxane backbone may be crosslinked with rubber-like polymers such as polybutadiene and polyisoprene, with the unsaturated fatty acid being incorporated in such a crosslinked product. This for example may be done to tune the water permeability of the final material as silicones typically have a rather open structure whilst such rubber-like polymers have a rather closed structure, such that the openness, i.e. the water permeability, of the crosslinked product can be tuned by the ratio of silicone and rubber-like polymer therein. In this manner, the properties of the material according to this embodiment may be optimized for cell/protein interaction to build good scaffolds. For example, where the elastomer is a silicone-based, e.g. PDMS-based, a material that is highly permeable to water and other compounds (e.g. drugs) is obtained that facilitates maximized perfusion of these compounds into the cells on the membrane of the fluidic device module 100. However, where real-time monitoring of drug consumption by cells is desirable, a polyene-based material such as a polybutadiene-based material that is non-permeable to water and drugs may be preferable, as the rate of perfusion of these compounds to the cells can be controlled using the density of apertures through the membrane.

In an embodiment, the polysiloxane may be formed from a multi-component starting material as previously explained in order to prevent premature cross-linking of the polysiloxane. Such multi-component starting materials are well-known per se; for example, such multi-component starting material kits are marketed by Wacker Chemie AG from Munich, Germany under the tradename Elastosil.

Regarding to unsaturated fatty acid 20, any suitable unsaturated fatty acid may be used for the purpose of cross-linking it with the elastomer 10. For example, the unsaturated fatty acid 20 may be selected from myristoleic acid, palmitoleic acid, sapienic acid, oleic acid, elaidic acid, vaccenic acid, linoleic acid, linoeladic acid, α-linolenic acid, arachidonic acid, eicospaentaenoic acid, erucic acid, undodecanoic acid and docosahexaenoic acid. Linoleic acid has been used in the examples of the present invention but it should be understood that this is by way of non-limiting example only and that other unsaturated fatty acids may be used instead. In the context of the present application, where reference is made to an unsaturated fatty acid 20, it is to be understood that this is intended to refer to any organic compound comprising a carboxylic acid head group covalently bound to an aliphatic tail comprising in between 10-30 carbon atoms and at least one carbon-carbon double bond in the aliphatic tail. The aliphatic tail may terminate in a non-aromatic ring structure, e.g. a five-membered or 6-membered cyclopentyl or cyclohexyl group, which may itself contain at least one double bond. A non-limiting example of an organic compound intended to fall within the above definition of an unsaturated fatty acid 20 is retinoic acid. Many other compounds in addition to the example compounds explicitly mentioned in the present application will be immediately apparent to the skilled person.

An example synthesis procedure for producing the bulk-modified elastomer from a polyene such as polybutadiene is given below (synthesis example 1).

### Synthesis example 1

Polybutadiene chunks as obtained from Lanxess AG, Cologne, Germany are inserted into a compounder or mix extruder. The chunks contain a peroxide such as Dicumylperoxide as a catalyst. An amount of linoleic acid (60-74% concentrated as obtained from Aldrich Chemistry) in a range of 1-5 wt% by weight of the polybutadiene chunks is mixed into the polybutadiene bulk by kneading at a temperature in the range of 110-150°C. The resulting extruded mixture is injected into a stainless steel mold defining a fluidic device and having an oxidized inner surface contacting the extruded mixture for 3-10 minutes at a temperature in a range of 150-200°C to yield a monolithic fluidic device module of a polybutadiene rubber cross-linked with the linoleic acid.

An example synthesis procedure for producing the bulk-modified elastomer from a silicone rubber is given below (synthesis example 2).

### Synthesis example 2

A 4.848 g sodium linoleate solution in a solution of water and isopropylalcohol (1:1 ratio of the solvents by weight) such that the weight fraction of the sodium linoleate in solution is 30% by weight is mixed into silicone component A (240g) of Elastosil LR 3040 from Wacker Chemie AG and vigorously stirred under vacuum conditions at room temperature to evaporate the water from the composition. The resultant mixture is mixed with component B (240g) of Elastosil LR 3040 from Wacker Chemie AG (including Pt-catalyst) and fed into an injection molding apparatus where the resultant mixture is injected into a stainless steel mold having an oxidized inner surface contacting the resultant mixture and defining a fluidic device module and molded for 10-60 seconds at a temperature in a range of 150-200°C to yield a monolithic fluidic device module of a silicone rubber cross-linked with the sodium linoleate in which the sodium linoleate is present in the polymer matrix in an amount of 1% by weight relative to the weight of the polymer matrix (based on starting material weights). Optionally, the molded product may be subsequently dropped in an aqueous HCl solution (pH 2-4) to convert at least the sodium carbonate groups at the surface of the molded product into free carboxylic acid groups.

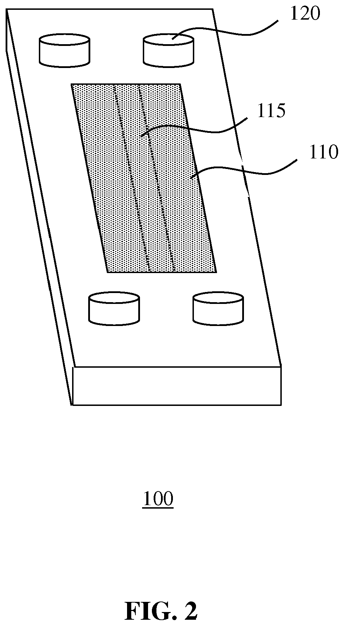

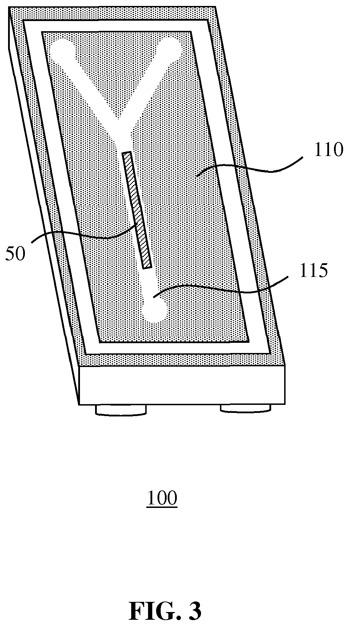

Such a bulk-modified elastomer may be molded in any suitable form to produce an article onto which cells may be directly cultured. Such articles may take any suitable shape. In a particular embodiment, the mold used in such an injection molding process may be shaped in the form of a fluidic device module such that a monolithic fluidic device module may be formed in a single step injection molding process. Due to the high density and homogeneous distribution of carboxylic acid groups or carboxylate groups on the external surface(s) of such a monolithic fluidic device module, such a device module can be used a straightforward manner as a cell-culturing substrate. The mold may be made of or have an inner surface coated with any suitable polar material, such as stainless steel, a metal or a metal oxide such as aluminium oxide in order to achieve the desired orientation of the carboxylic acid or carboxylate groups of the bulk-modified elastomer on at least one of its external surfaces. A typical example of a fluidic device module 100 is schematically depicted in FIG. 2 and FIG. 3, in which perspective views of opposing major surfaces of the fluidic device module 100 are schematically depicted. The fluidic device module 100 may comprise a membrane 110 over which at least one flow channel 115 extends. Preferably, the fluidic device module 100 comprises opposing flow channels spatially separated by the membrane 110. At least the membrane 110 is made of the bulk-modified elastomer 30 although in a preferred embodiment the entire fluidic device module 100 is made of the bulk-modified elastomer 30, thereby yielding a monolithic fluidic device module 100.

The fluidic device module 100 may comprise one or more septums 120 through which such flow channels 115 may be accessed. Each flow channel 115 preferably is accessible through a dedicated set of septums 120, such that cross-contamination between multiple flow channels 115 is avoided. As explained above, an exposed surface of the fluidic device module 100, e.g. the membrane 110, may be used to directly bind a cell culture, such that cells may be cultured (proliferated) on the biocompatible surface.

The membrane 110 may comprise a plurality of holes or grooves such that liquid nutrition and solutions with compounds such as potential drugs may reach both sides of the membrane and cells/tissue, which holes or grooves may be formed in any suitable manner, e.g. through laser cutting. Such holes or grooves in the membrane 110 of the fluidic device module 100 preferably are in the same order as the typical diameter of the cells to be immobilized on the membrane 110, with the pitch between such holes or grooves not being particularly critical.

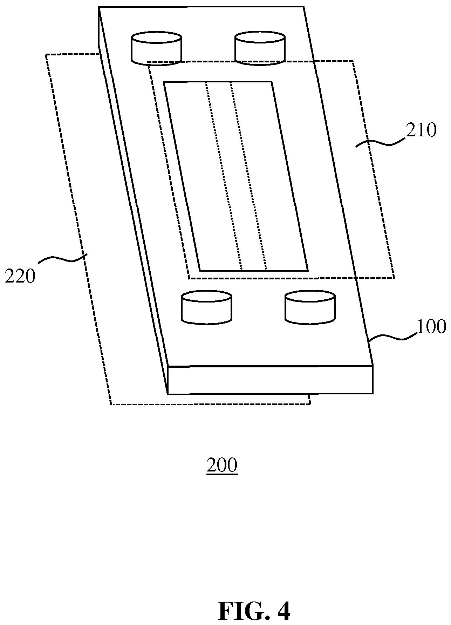

A fluidic device 200 as schematically depicted in FIG. 4 may be formed from such a fluidic device module 100 by the provision of a pair of cover plates 210, 220, which cover plates fluidly seal the fluidic device module 100 such that fluids passed through the one or more flow channels 115 of the fluidic device module 100 cannot leak out of the fluidic device 200.

In an embodiment, the cover plates 210, 220 are arranged such that the cover plate 210 covers a first major surface of the fluidic device module 100, thereby sealing a first fluidic channel 115 of the fluidic device module and the other cover plate 220 covers a second major surface of the fluidic device module 100, thereby sealing a second fluidic channel 115 of the fluidic device module. The cover plates 210 and 220 may be made of any suitable material, e.g. glass or a plastic material. Preferably, the cover plates are stretchable and flexible to ensure a good fit with the fluidic device module 100 whilst retaining the flexibility of the fluidic device module 100 in the fluidic device 200. Such a fluidic device 200 may be deployed as an organ on chip as will be readily understood by the skilled person.

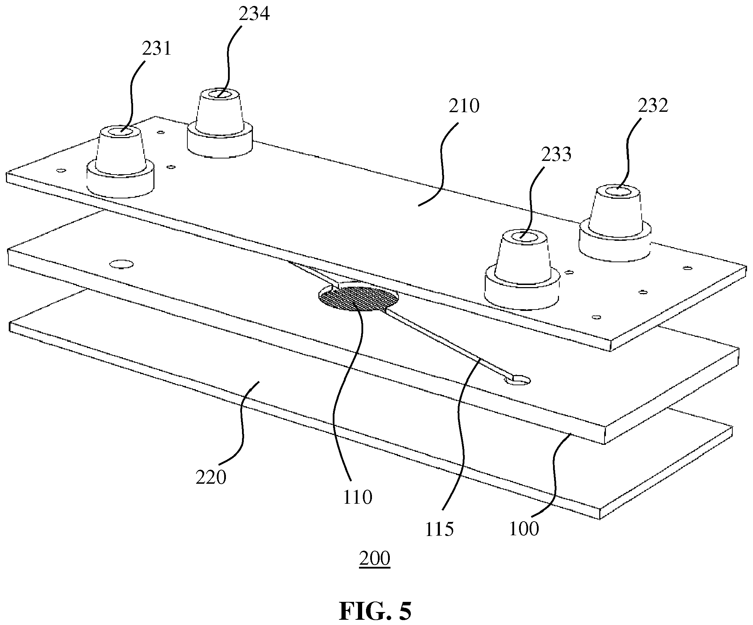

FIG. 5 schematically depicts an example embodiment of a fluidic device 200 in which the device comprises a fluidic device module 100 according to the present invention in between two rigid cover plates 210 and 220. The upper plate 210 comprises a first inlet 231 and a first outlet 232 in fluid connection with a lower channel (not visible) underneath the membrane 110 in the fluidic device module 100 such that the membrane 110 is in fluid contact with a fluid passing through this lower channel. The upper plate 210 further comprises a second inlet 233 and a second outlet 234 in fluid connection with an upper channel 115 over the membrane 110 such that the membrane 110 is in fluid contact with a further fluid passing through this upper channel 115. For example, the fluid passing through the lower channel may comprise drugs to be tested on the cell cultures attached to the membrane 110 as explained in more detail below, which can reach the cell cultures through the membrane 110 owing to the fact that the membrane 110 is made porous, e.g. by laser drilling holes or grooves in the membrane 110 as previously explained. Alternatively, such holes or grooves may be formed in the membrane 110 when forming the fluidic device module 100 in the polar mold. This has the advantage that a regular pattern of such fluid passages (pores) may be formed through the membrane 110. The further fluid passing through the upper channel 115 may be in direct contact with such cell cultures and provide such cell cultures with nutrients. The inlets 231, 233 and the outlets 232, 234 in some embodiments are septa through which fluids may be hydraulically pumped over the membrane 110 through the aforementioned channels, e.g. by pressing the septa. Alternatively, the inlets 231, 233 and the outlets 232, 234 may be ferrules to which tubing may be attached, e.g. clamped, which for instance is useful when the fluidic device 200 is used as a well plate or the like in a lab on chip system.

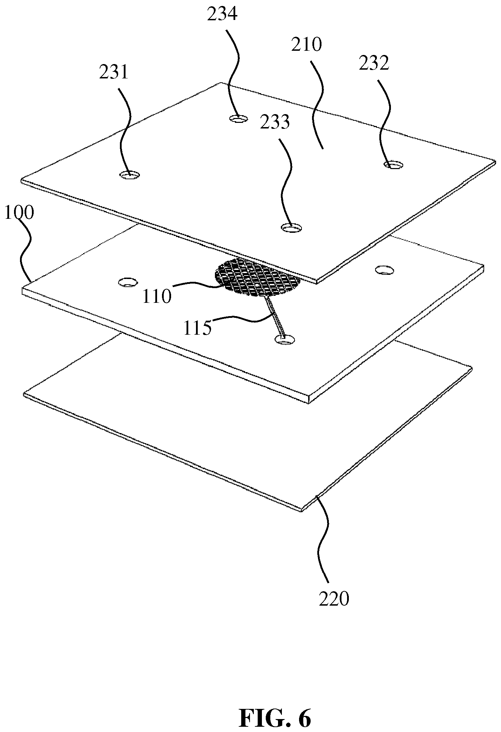

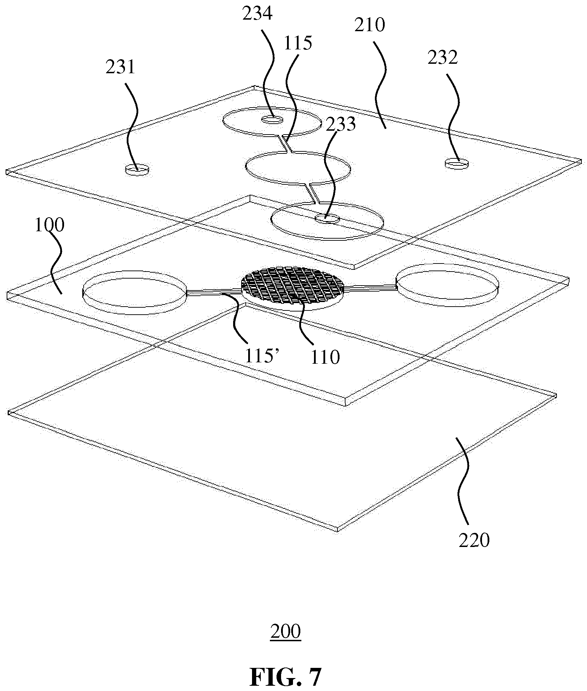

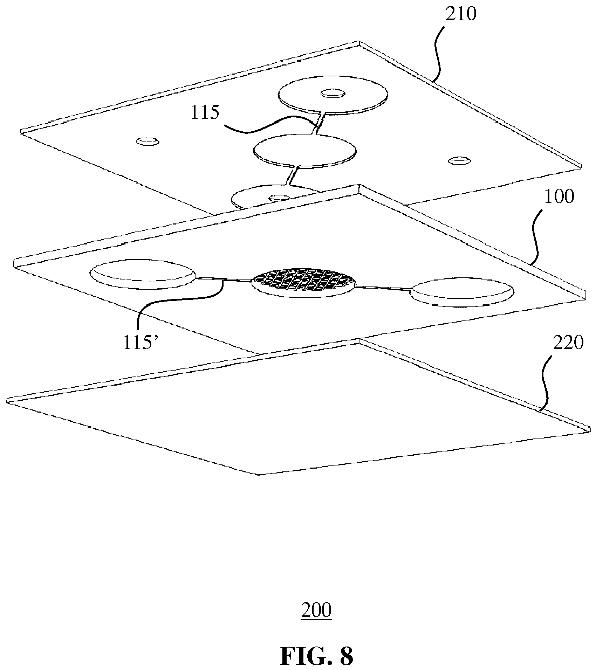

In an embodiment schematically depicted in FIG. 6, the upper and lower channels 115 are incorporated in the (monolithic) fluidic device module 100 (only one of the channels is shown), e.g. when molding the fluidic device module 100. Alternatively, at least one of the upper and lower channels may be formed in one of the cover plates. FIG. 7 schematically depicts a perspective top view and FIG. 8 schematically depicts a perspective bottom view of an example embodiment of a fluidic device 200 in which the upper channel 115 is formed in the upper cover plate 210 whilst the lower channel 115' is formed in the fluidic device module 100. It is of course equally feasible to form the lower channel 115' in the bottom cover plate 220 as will be readily understood by the skilled person. The fluidic device 200 such as the device shown in FIG. 7 and FIG. 8 for example may be used as a microwell in a lab on chip device in which a plurality of such microwells may be clamped or otherwise secured to facilitate parallel testing of a plurality of different samples within a single lab on chip device, in which each microwell typically comprises one of these samples.

Upon immobilizing a cell culture 50 in such a fluidic device 200 and proliferating this cell culture, the fluidic device 200 may be used in methods in which the immobilized proliferated cell culture 50 is exposed to a fluid comprising a compound of interest, which fluid may be passed through a flow channel 115 of the fluidic device 200 in order to expose the immobilized cell culture 50 to the compound of interest in the fluid and monitor the reaction of the immobilized cell culture 50 to such exposure. Such a method for example may be deployed in an oncology setting where the immobilized cell culture 50 may be exposed to a drug, e.g. a chemotherapy drug, in order to monitor the response of the immobilized cell culture 50 to such a drug. To this end, the immobilized cell culture 50 may include tumor cells in order to test the efficacy of the drug and/or may include healthy cells in order to test the toxicity of the drug on healthy tissue.

Such a drug testing method typically involves providing a fluidic device module 100 in accordance with one or more embodiments of the present invention, e.g. providing a monolithic fluidic device module 100 and applying a harvested cell culture to at least the membrane 110 where the cell culture can directly bind to the carboxylic acid or carboxylate groups on this exposed surface to obtain a prepared fluidic device module 100 from which a fluidic device 200, e.g. an organ on chip, is formed as previously explained. The cell culture within the fluid device 200 may be fed through one of a pair of flow channels 115 of the fluidic device 200 in order to keep the cell culture alive, whilst the cell culture may be exposed to a drug to be tested through the other of the pair of flow channels 115 of the fluidic device 200, with the drug testing method typically being completed by monitoring the response of the cell culture to the drug to be tested. This for example may be achieved by disassembling the fluidic device 200 by removing the cover plates 210, 220 and slicing the fluid device module 100 such that a slice of this module including a portion of the cell culture is obtained, which slice may be investigated under a microscope or the like in order to investigate the effects of the drug under test on the cell culture. During such investigations, the cell culture is typically stained, treated with formalin and fixated in a fixating agent such as paraffin to facilitate such microscopic investigation. Such staining, treating and fixating of the cell culture may be deployed within the fluidic device 200, e.g. by passing the appropriate chemical agents through a fluidic channel 115 of the fluidic device 200 in which the cell culture is exposed.

In an alternative embodiment, such disassembly of the fluidic device 200 can be avoided and the monitoring of the response of the cell culture to one or more compounds such as a drug to be tested can be achieved in real time using confocal microscopy. In particular, where embodiments of the fluidic device 200 comprise transparent cover plates 210, 220, e.g. glass plates or polymer plates, confocal microscopy may be used to monitor the cell culture within an assembled fluidic device 200. This is made possible through the fact that at least the bottom cover plate 220 can be kept thin, for example having a thickness in a range of 150-300 µm, with the distance between the membrane 110 of the fluidic device module 100 carrying the cell culture and the bottom cover plate 220 being less than 200 µm such that the focal length limits of confocal microscopy (typically around 500 µm from the object to be investigated to the lens objective). To this end, the membrane 110 may have a thickness in a range of 10-100 µm, such as a thickness of 20-30 µm. The thickness of the upper cover plate 210 is not critical; any suitable thickness may be contemplated, such as a thickness of 300 µm - 3 mm.

The evaluation of the cell culture within the fluidic device 200 using confocal microscopy is particularly suitable where the cell spheroids have a diameter of less than 500 µm, e.g. about 200 µm in order to ensure that the overall focal length of the optical path facilitates the capture of a sharp image with the confocal microscope. For larger spheroids, the fluidic device 200 may have to be dismantled such that the membrane 110 including the spheroids can be pressed onto a glass cover slip for positioning in the optical objective of the confocal microscope.

It is furthermore noted that the aforementioned dimensions of the fluidic device 200 typically apply during the real-time monitoring of the cell culture within the fluidic device 200. In embodiments in which the fluidic device 200 is sliced as previously explained, e.g. for digital pathology, the dimensions of the fluidic device 200 are less critical as long as the formalin and paraffin fixation can be performed thereon.

As will be readily understood by the skilled person, such drug testing methods may significantly differ in terms of protocol, e.g. drug dosages, administration frequencies, and so on. It should be understood that the drug testing method of the present invention is not limited to a particular protocol as long as such methods may be deployed using a fluidic device 200 according to an embodiment of the present invention in which cells are cultured directly onto an exposed surface, e.g. the membrane 110, of the device.

At this point, proof of concept of the cultivation of mammalian cells directly onto the bulk-modified elastomer of the present invention will be provided in the form of the following experimental evidence.

### General procedure:

Samples were cleaned by washing the samples in Iso-propanol for 5 minutes. After drying, the samples were placed in a 24 well plate. A cell culture of immortalized human prostate fibroblasts (WPMY-1 cells, ATCC CRL-2854) was provided in DMEM (Dulbecco's Modified Eagle Medium, Thermofisher) with 10% FBS (Feutal Bovine Serum), 1% Penicillin/Streptomycin and 1% Glutamax. A total amount of 2.0 x 10^5 cells were seeded on all samples, and placed in an incubator overnight at 37°C and under an atmosphere of 5% CO₂. Images of the thus generated cell cultures were made using a Leica Inversed bright-field microscope at 100 times magnification.

Example 1: The WPMY-1 cell culture was developed in accordance with the general procedure as described above on a silicone elastomer bulk modified in accordance with the teachings of the present invention with 0.5 wt% linoleic acid. To this end, synthesis example 2 was amended by replacing the 4.848 g of sodium linoleate with 2.41 g of linoleic acid to obtain the bulk modified silicone elastomer used in this example.

Comparative Example 1: The WPMY-1 cell culture was developed in accordance with the general procedure as described above on the same silicone elastomer as example 1 but without bulk modification.

Example 2: The WPMY-1 cell culture was developed in accordance with the general procedure as described above on the bulk modified silicone elastomer prepared in accordance with synthesis example 2 (i.e. containing 1.0% by weight of sodium linoleate).

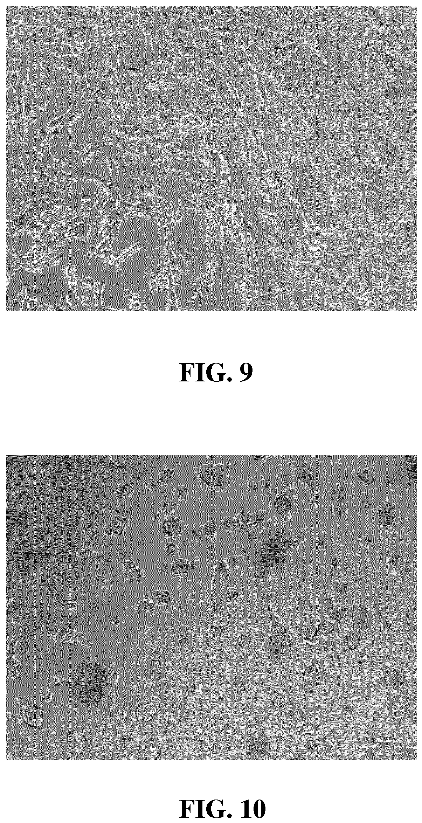

FIG. 9 shows the resulting cell culture of example 1 after the overnight cultivation. It can be clearly seen in this image that the cells show good adherence to the surface of the linoleic acid-functionalized silicone.

FIG. 10 shows the resulting cell culture of comparative example 1 after the overnight cultivation. It can be clearly seen in this image that the cells show minimal adherence to the surface of the unfunctionalized silicone elastomer.

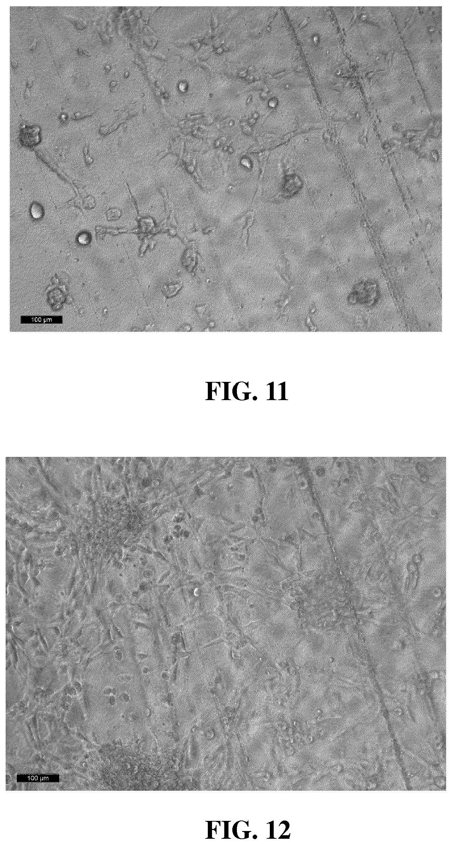

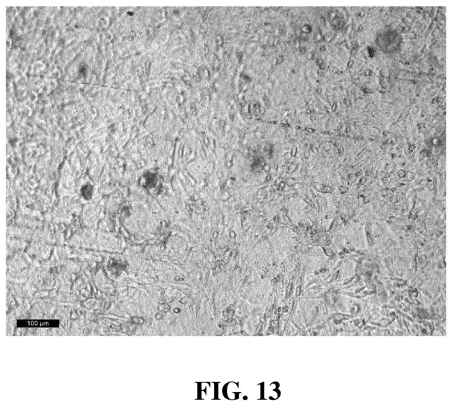

FIG. 11 shows the resulting cell culture of example 2 after the overnight cultivation. It can be clearly seen in this image that the cells show good adherence to the surface of the sodium linoleate-functionalized silicone. FIG. 12 shows the cell culture of example 2 after culturing for 2 days and FIG. 13 shows the cell culture of example 2 after culturing for 7 days. Ongoing cell proliferation can clearly be recognized from these images, which demonstrates that mammalian cell material can be directly cultured and proliferated onto the bulk-functionalized elastomers of the present invention.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A material for culturing cells, the material containing a bulk-modified elastomer having a Shore hardness (DIN EN ISO 868) in a range of Shore00 20 to Shore A 80 and comprising a plurality of fatty acid moieties covalently bound to the elastomer bulk, wherein the carboxylic acid groups of said moieties are available on an external surface of said material to provide said binding, and wherein the bulk-modified elastomer is obtained by:
forming a composition comprising a vinyl-functionalized or a hydride-functionalized elastomer or at least one precursor thereof, a free of saponified unsaturated fatty acid in a range of 0.5 - 5 % by weight of the total weight of the a vinyl-functionalized or a hydride-functionalized elastomer or at least one precursor thereof and a cross-linking catalyst in a mold having a polar inner surface; and
bulk-modifying the vinyl-functionalized or the hydride-functionalized elastomer by covalently binding the free or saponified unsaturated fatty acid to the elastomer bulk in said mold by a cross-linking reaction between a vinyl group or a hydride group of the elastomer and an unsaturated carbon-carbon bond of the unsaturated fatty acid to obtain the material.

2. The material of claim 1, wherein each of the fatty acid moieties is covalently bound to the elastomer bulk through a cross-linking reaction between a vinyl or hydride functional group of the elastomer and an unsaturated carbon-carbon bond of an unsaturated fatty acid.

3. The material of claim 2, wherein the unsaturated fatty acid is selected from myristoleic acid, palmitoleic acid, sapienic acid, oleic acid, elaidic acid, vaccenic acid, linoleic acid, linoeladic acid, α-linolenic acid, arachidonic acid, eicospaentaenoic acid, erucic acid and docosahexaenoic acid.

4. The material of any of claims 1-3, wherein the elastomer comprises a polybutadiene backbone or a silicone backbone.

5. The material of any of claims 1-4, wherein the composition comprises the free or saponified unsaturated fatty acid in a range of 0.5 - 2 % by weight of the total weight of the a vinyl-functionalized or a hydride-functionalized elastomer or at least one precursor thereof.

6. The material of any of claims 1-5, wherein the polar inner surface of said mold is a metal oxide inner surface.

7. A fluidic device module including a flow channel extending over a membrane comprising the material of any of claims 1-6.

8. The fluidic device module of claim 7, comprising a first major surface comprising a first recessed structure defining a first flow channel and a second major surface opposing the first major surface and comprising a second recessed structure defining a second flow channel; wherein the membrane separates the first flow channel from the second flow channel.

9. The fluidic device module of claim 7 or 8, wherein the fluidic device is a monolithic fluidic device.

10. A fluidic device comprising the fluidic device module of any of claims 7-9 and a pair of cover plates for fluidly sealing the fluidic device module.

11. The fluidic device of claim 10, wherein the cover plates are arranged such that one of said cover plates covers the first major surface, thereby sealing the first fluidic channel and the other of said cover plates covers the second major surface, thereby sealing the second fluidic channel.

12. A method of culturing cells, comprising:
providing a molded article formed from the material of any of claims 1-5, said article comprising the external surface of the material as one of its exposed surfaces; and
culturing said cells directly on said exposed surface of the article.

13. The method of claim 12, wherein said article comprises the fluid device module of any of claims 7-9.

14. A drug testing method comprising:
providing a fluidic device module according to claim 8;
applying harvested cells to a surface of the membrane of the fluidic device module;
culturing the harvested cells on said surface;
forming a fluidic device with the prepared fluidic device module;
feeding the cultured cells through one of the pair of flow channels of the fluidic device;
exposing the cultured cells to a drug to be tested through the other of the pair of flow channels of the fluidic device; and
monitoring the response of the cultured cells to the drug to be tested.

15. The drug testing method of claim 14, wherein monitoring the response of the proliferated cell culture to the drug to be tested comprises:
immobilizing and fixating the cultured cells within the fluid device and slicing the fluid device module to obtain a slice for microscopic evaluation, said slice comprising at least a portion of the immobilized and fixated proliferated cell culture; or
monitoring the cultured cells within the fluid device using confocal microscopy.
